# EUROPÄISCHE PATENTSCHRIFT

(11) **EP 0 633 028 B1**
(45) Veröffentlichungstag und Bekanntmachung des Hinweises auf die Patenterteilung: **17.11.1999**
(21) Anmeldenummer: 94112145.1
(22) Anmeldetag: 18.09.1990
(51) Int. Cl.: A61K 39/02, A61K 39/40, C12P 21/08

(54) **Impfstoff gegen die Lyme-Krankheit**
Vaccine against lyme disease
Vaccin contre la maladie de lyme

(30) Priorität: 19.09.1989 DE 3931236; 17.05.1990 DE 4015911
(43) Veröffentlichungstag der Anmeldung: 11.01.1995
(62) Teilanmeldung aus: 90117943.2
(73) Patentinhaber: Max-Planck-Gesellschaft zur Förderung der Wissenschaften e.V., 80539 München (DE); Deutsches Krebsforschungszentrum Stiftung des öffentlichen Rechts, 69120 Heidelberg (DE)
(72) Erfinder: Simon, Markus, M., Dr., D-79102 Freiburg (DE); Schaible, Ulrich E., St. Louis, MO 63105 (US); Eichmann, Klaus, Prof.Dr., D-79104 Freiburg (DE); Kramer, Michael, Dr., D-69120 Heidelberg (DE); Reinhard, Wallich, Dr., D-69118 Heidelberg (DE)
(74) Vertreter: Weiss, Wolfgang, Dipl.-Chem. Dr.

(56) Entgegenhaltungen:
- EP-A- 0 252 641
- WO-A-90/04411
- WO-A-91/09870
- WO-A-92/00055
- PROCEEDINGS OF THE NATIONAL ACADEMY OF SCIENCES OF USA., Bd.87, Mai 1990, WASHINGTON US Seiten 3768 - 3772 ULRICH E. SCHAIBLE ET AL. 'MONOCLONAL ANTIBODIES SPECIFIC FOR THE OUTER SURFACE PROTEIN A (OSPA) OF BORRELIA BURGDORFERI PREVENT LYME BORRELIOSIS IN SEVERE COMBINED IMMUNODEFICIENCY (SCID) MICE'
- B.WILSKE ET AL: "Antigenic variability of Borrelia burgdorferei", ANN. N. Y. ACAD. S., 1988, Band 539, Seiten 126 - 143

## Beschreibung

Die Lyme-Borreliose ist die häufigste, von Zecken übertragene Infektionskrankheit in den gemäßigten Breiten. Sie wird durch die Spirochäte Borrelia burgdorferi hervorgerufen, die vor allem durch Zecken des Stamms Ixodes auf den Menschen übertragen wird. Die Krankheit ist eine chronische progressive Infektion, die viele Organe, wie die Haut, das zentrale und periphere Nervensystem, das Herz, die Leber, die Niere und das musculosketale System befällt. Da eine zuverlässige Behandlung dieser Krankheit durch Therapie mit Antibiotika schwierig ist, werden gegenwertig große Anstrengungen unternommen, den Erreger selbst und die Immunantwort des Wirts auf Infektion mit B. burgdorferi zu erforschen.

D. Wilske et al. (Ahn. N. Y. Acad. S. 539, Seiten 126-143 (1988)) beschreiben eine immunchemische Analyse von B. burgdorferi Proteinen unter Verwendung von monoklonalen und polyklonalen Antikörpern im Western Blot. Es wird über eine Variabilität der Oberflächenantigene OspA und OspB in nordamerikanischen und europäischen Stämmen berichtet. Die Oberflächenantigene OspA und OspB werden ausdrücklich nicht als bedeutendes Immunogen bei der menschlichen Erkrankung bezeichnet.

In WO 90/04411, die für die Vertragsstaaten AT, BE, CH/LI, DE, DK, FR, GB, IT, LU, NL und SE einen Stand der Technik im Sinne von Art. 54(3) EPÜ darstellt, werden immunogene Fraktionen aus B. burgdorferi beschrieben, die mit Hilfe eines Reinigungsverfahrens isoliert werden. Die gereinigten Fraktionen sind frei von Zellwand- und Flagellinkomponenten und zeigen dieselbe immunogene Aktivität wie Extrakte aus ganzen B. burgdorferi Zellen. Diese Fraktionen können zur Immunisierung gegen die und zur Diagnose der Lyme-Krankheit verwendet werden. Weiterhin wird ein DNA-Fragment offenbart, das für ein B.burgdorferi OspA Protein kodiert.

Bei von der Lyme-Krankheit betroffenen Personen wird zwar ein hoher Titer an Antikörpern gegen B. burgdorferi festgestellt, der aber keinen Schutz gegen die Infektion bewirkt. Es wird angenommen, daß der Erreger sehr rasch aus der Blutbahn in das Gewebe übertritt und dort für das Immunsystem nicht unmittelbar erreichbar ist. Dies würde bedeuten, daß ein Schutz durch Antikörper nur unmittelbar nach Beginn der Infektion, solange also die Erreger sich noch in der Blutbahn befinden, möglich ist.

Die Tatsache, daß eine natürliche Infektion mit B. burgdorferi in verschiedenen Tierarten gefunden wurde, hat zu dem Versuch geführt, Labormodelle für die Lyme-Krankheit zu etablieren. Dies gelang auch mit begrenztem Erfolg. So wurde bei Experimenten, welche die Induzierung einer für B. burgdorferi spezifischen Immunantwort in der Maus zum Ziel hatten, gefunden, daß die Infektion von Inzucht-Mausstämmen mit einem schon lange Zeit kultivierten B. burgdorferi-Isolat zu mäßigen, aber signifikanten pathomorphologischen Veränderungen in verschiedenen Organen wie dem Gehirn, dem Herz, den Lungen und den Nieren führte, die vergleichbar mit denjenigen waren, die bei Patienten mit Lyme-Krankheit zu beobachten sind (Schaible et al., (1988) Infect. Immun. 1, 41). Die Ausbildung eines ernsteren Krankheitsbildes bei Tieren wurde vermutlich entweder durch die Immunabwehr des Wirts und/oder durch die reduzierte Virulenz von für einen längeren Zeitraum in vitro kultivierten Spirochäten (Johnson et al., (1984), J. Clin. Microbiol. 20, 747; Schwan et al., (1988), Infect. and Immun. 56, 1837) verhindert.

Die der Erfindung zugrundeliegende Aufgabe ist es, einen wirksamen Impfstoff gegen die Lyme-Krankheit bereitzustellen. Dazu ist jedoch zunächst die Entwicklung eines geeigneten tierischen Labormodells erforderlich. Es wird nun vorgeschlagen, daß ein Maus-Stamm ohne funktionsfähige T- und B-Zellen, die sogenannte Scid-Maus (Bosma et al., (1983) Nature 10, 52), als Versuchstier dienen kann, da Scid-Mäuse bei Infektion mit einem pathogenen B. burgdorferi-Isolat eine multisystemische Krankheit und zwar hauptsächlich Polyarthrithis und Carditis, entwickeln. Durch dieses Tiermodell wird es erst möglich, die Wirkung von Impfstoffen gegen die Lyme-Krankheit zu erproben.

Ein Gegenstand der Erfindung für die Vertragsstaaten ES und GR ist ein Verfahren zur Gewinnung eines Impfstoffs gegen die Lyme-Krankheit, wobei man Versuchstiere mit dem 31 kD Antigen (OspA) oder/und dem 34 kD Antigen (OspB) von B. burgdorferi immunisiert und aus dem immunisierten Versuchstier auf übliche Weise protektive, polyklonale oder monoklonale Antikörper gegen OspA oder/und OspB gewinnt.

Ein Gegenstand der Erfindung für die Vertragsstaaten AT, BE, CH/LI, DE, DK, FR, GB, IT, LU, NL und SE ist ein Verfahren zur Gewinnung eines Impfstoffs gegen die Lyme-Krankheit, wobei man Versuchstiere mit dem 31 kD Antigen (OspA) oder/und dem 34 kD Antigen (OspB) von B.burgdorferi) ZS7 (DSM 5527) und aus dem immunisierten Versuchstier auf übliche Weise protektive, polyklonale oder monoklonale Antikörper gegen OspA oder/und OspB gewinnt.

Dabei wird ein passiver Impfstoff gegen die Lyme Krankheit erhalten, der einen oder mehrere für das 31 kD Antigen (OspA) oder/und das 34 kD Antigen (OspB) von B. burgdorferi, insbesondere OspA oder/und OspB von B. burgdorferi der Stämme B31 (ATCC 35210) oder/und ZS7 (hinterlegt in der Deutschen Sammlung von Mikroorgansimen und Zellkulturen GmbH am 19.09.1989, Zugangsnummer DSM 5527) spezifische monoklonale Antikörper enthält. Bevorzugt ist ein Impfstoff, der einen Antikörper der Klasse IgG, besonders bevorzugt der Subklasse IgG2b oder IgG1, enthält. Die Verabreichung des Antikörpers bewirkt überraschenderweise im Gegensatz zur Verabreichung eines anderen Antikörpers, z.B. gegen das 41 kD Oberflächenantigen von B. burgdorferi (Flagellin) bei immundefizienten Versuchstieren, vorzugsweise Scid-Mäusen, die mit lebensfähigen pathogenen B. burgdorferi, vorzugsweise B. burgdorferi ZS7 infiziert wurden, daß die Ausbildung von Arthritis, Carditis und Hepatitis vollständig oder zumindest weitgehend verhindert wird.

Gegebenenfalls kann der erfindungsgemäße Impfstoff mit dem Antikörper als Wirkstoff auch noch übliche Träger-, Füll- und Hilfsstoffe enthalten.

Weiterhin beinhaltet die Erfindung auch ein Verfahren zur Gewinnung eines passiven Impfstoffs gegen die Lyme-Krankheit aus Lymphozyten oder Milzzellen eines Versuchstieres, vorzugsweise einer Maus, die mit B. burgdorferi Organismen oder Teilen davon, vorzugsweise mit kompletten B. burgdorferi B31 oder/und ZS7 Organismen, immunisiert ist, wobei man aus dem Lymphozyten oder Milzzellen des immunisierten Tieres durch Zellfusion ein Hybridom gewinnt, das einen erfindungsgemäßen monoklonalen Antikörper produziert.

Durch das erfindungsgemäße Verfahren wurde eine Hybridoma-Zellinie (hinterlegt in der European Collection of Animal Cell Cultures am 13.09.1989, Zugangsnummer ECACC 89 09 1302) gewonnen, die einen Antikörper LA-2 gegen OspA (IgG2b) produziert. Weiterhin wurden die den Antikörper LA-26.1 gegen OspA (IgG1) produzierende Hybridoma-Zellinie ECACC 90050406 (hinterlegt am 4.05.1990), sowie die die Antikörper LA-25.1 bzw. LA-27.1 gegen OspB (IgG2b bzw. IgG1) produzierenden Hybridoma-Zellinien ECACC 90050405 bzw. ECACC 90050407 (hinterlegt am 4.05.1990) gewonnen.

Zur Immunisierung kann ein Antigen verwendet werden, welches die gesamte Aminosäuresequenz von OspA bzw. OspB oder auch nur eine immunogen wirkende Teilsequenz (immunogenes Epitop) von OspA bzw. OspB enthält. Potentiell immunogene Epitope dieser Proteine kann ein Fachmann ohne Schwierigkeiten durch eine Strukturanalyse des OspA-Protein (z.B. Chou-Fassmann Analyse) ermitteln und dann experimentell auf ihre Wirksamkeit testen.

Insbesondere ist auch ein rekombinantes Antigen geeignet, wobei sich die für das Antigen kodierende DNA-Sequenz auf einem rekombinanten Vektor, vorzugsweise einem prokaryontischen Vektor befindet, der geeignet zur Proteinexpression ist.

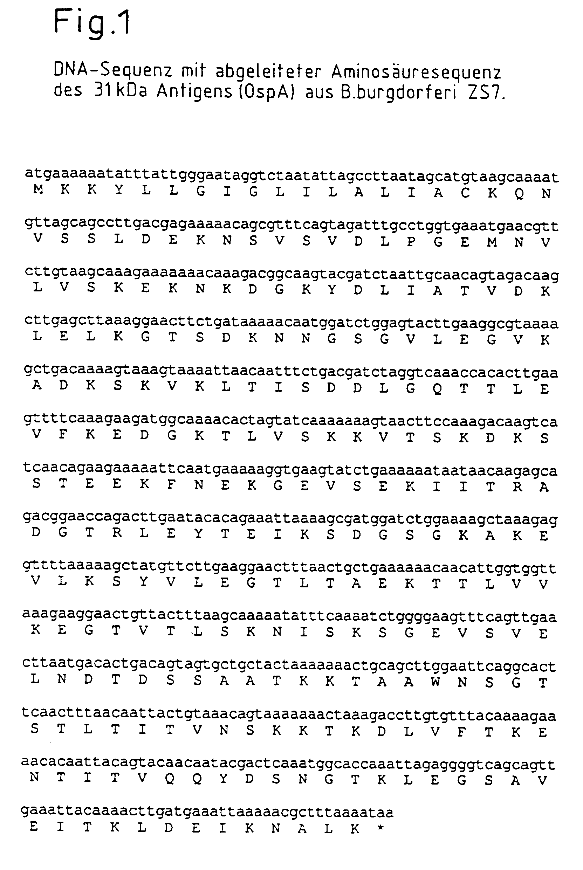

Insbesondere geeignet ist ein Antigen aus B.burgdorferi ZS7, das die in Abb. 1 gezeigte Aminosäuresequenz oder ein immunogenes Epitop aus dieser Sequenz enthält. Dieses Antigen wird kodiert von einer rekombinanten DNA, welche (1) die in Abb. 1 gezeigte, (2) eine, ihr im Rahmen der Degeneration des genetischen Codes entsprechende Nukleinsäuresequenz oder (3) eine mit einer Sequenz aus (1) oder/und (2) unter stringenten Bedingungen hybridisierende Sequenz enthält, die für das 31 kD Antigen von B. burgdorferi ZS7 oder ein immunogenes Epitop davon kodiert. Der Begriff "stringente Hybridisierungsbedingungen" ist dabei wie in Maniatis et al., Molecular Cloning. A Laboratory Manual (1982), Cold Spring Harbor Laboratory, New York, zu verstehen.

Besonders bevorzugt ist ein erfindungsgemäßes Antigen, das ein rekombinantes Nicht-Fusionsprotein oder β-Galactosidase-Fusionsprotein ist.

Die für das Antigen kodierende DNA-Sequenz befindet sich vorzugsweise auf einem rekombinanten Vektor, der eine oder mehrere Kopien einer rekombinanten DNA enthält. Der Vektor kann ein prokaryontischer oder/und eukaryontischer Vektor sein, er ist vorzugsweise ein prokaryontischer Vektor. Der rekombinante Vektor kann in der Wirtszelle extrachromosomal vorliegen (z.B. Plasmid) oder er kann sich auch in das Genom der Wirtszelle integrieren (z.B. Bakteriophage Lambda). Vorzugsweise ist der Vektor ein Plasmid. Besonders bevorzugt ist der rekombinante Vektor pZS-7/31-2 (DSM 5528).

Es konnte in der Tat gezeigt werden, daß die Verabreichung von nativem bzw. rekombinantem OspA in normalen Mäusen die Bildung protektiver Antikörer induziert, die nach passivem Transfer in Scid-Mäusen diese gegen die Lyme-Borreliose schützen. Insbesondere findet man, daß rekombinantes OspA eine mit nativem OspA vergleichbare protektive Immunantwort induziert und daher einen vielversprechenden Kandidaten für eine Vakzine gegen die Lyme-Borreliose im Menschen darstellt.

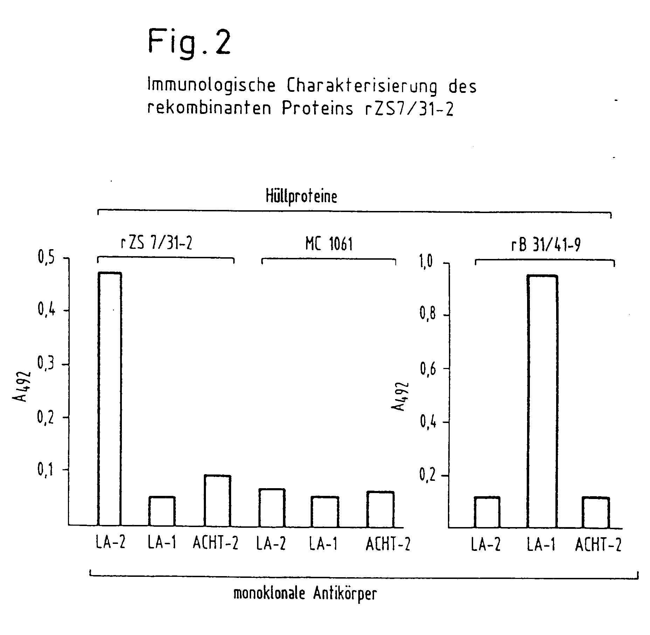

Die Verdeutlichung der Erfindung erfolgt durch nachfolgende Beispiele und die Abbildungen 1 und 2.

Es zeigen:
- Abb. 1: die DNA- und Aminosäuresequenz des 31 kD Antigens (OspA) aus B. burgdorferi ZS7,
- Abb. 2: die immunologische Charakterisierung des rekombinanten Proteins rZS7/31-2.

### Beispiel 1

### Induzierung von Arthritis, Carditis und Hepatitis in Scid-Mäusen durch Infektion mit B. burgdorferi Stamm ZS7.

### Behandlung der Mäuse mit B. burgdorferi

Erwachsenen Mäusen der Stämme C.B-17 Scid (homozygot für die Scid-Mutation) und C.B-17 wurden 1x10⁵, 5x10⁵, 1x10⁶ oder 1x10⁸ lebensfähige oder abgetötete (UV-Strahlung) B. burgdorferi Organismen subkutan in die Schwanzwurzel injiziert.

### Isolierung von B. burgdorferi aus Zecken und Mäusen

Die Untersuchungen wurden mit dem schon seit langem kultivierten B. burgdorferi Stamm B31 (ATCC 35210) und dem frischen Isolat B. burgdorferi ZS7 (DSM 5527), das aus einer weiblichen Ixodes rizinus Zecke isoliert wurde, durchgeführt. Alle B. burgdorferi Stämme wurden in modifiziertem Kelly's Medium kultiviert (Barbour et al., (1983) Switzerland. Curr. Microbiol. 8, 123). B. burgdorferi Organismen, die aus dem Mitteldarm von mit Ethanol sterilisierten Zecken oder aus Blut von infizierten Mäusen gewonnen wurden, wurden anfangs in Kelly's Medium unter Zusatz von 8 µg/ml Kanamycin und 230 µg/ml Fluoruracil kultiviert (Johnson et al., (1984) J. Clin. Microbiol. 1, 81).

### Serologische Tests

Die Detektion von B. burgdorferi spezifischen Antikörpern wurde in einem konventionellen ELISA-Verfahren durchgeführt (Justus et al., (1988) Wehrmed. Mschr. 32, 263). Die Standardkurve für den Gehalt an Immunglobulin (Ig) wurde durch Beschichtung einer Schale mit Anti-Maus Ig (1:500 Verdünnung der Serumlösung von Paesel, Frankfurt, BRD) und Titration des Gesamt-Maus IgG-oder IgM-Gehalts (Calbiochem., LaJolla, USA) erhalten. Gesamtserum IgM und IgG wurde ähnlich gemessen. Die Konzentration von B. burgdorferi spezifischen IgM- oder IgG-Antikörpern ist in µg Ig/ml Serum angegeben.

### Immunfluoreszenz und Giemsa-Anfärbung

50 µl Blut wurden in einen Hematocrit-Röhrchen pipettiert (Becton und Dickinson, Heidelberg, BRD) und bei 5.000 g in einer Hematocrit-Zentrifuge (ECCO, BRD) zentrifugiert. Die Röhrchen wurden an der Interphase zwischen Serum und Erythrozyten aufgeschnitten und 5 µl des Serums wurden auf Objektträger aufgetragen (Superior, Bad Mergentheim, BRD). Die mit den Serumproben beladenen Objektträger wurden an der Luft getrocknet und in 100 % Ethanol eine Minute lang bei -20°C fixiert. Nach einstündiger Inkubation mit Kaninchen-Anti B. burgdorferi Hyperimmunserum (1:100 Verdünnung) bei Raumtemperatur wurden die Objektträger fünfmal in PBS gewaschen und dann mit FITC konjugiertem Ziegen-Antikaninchen-Antiserum (1:20 Verdünnung, Jackson Lab., West Grove, USA) eine Stunde lang angefärbt. Die Objektträger wurden gewaschen und in Kaiser's Glycerin-Gelatine (Merck, Darmstadt, BRD) eingebettet und sofort fluoreszenzmikroskopisch untersucht. Unbehandelte Bluttropfen wurden an der Luft getrocknet, in Methanol fixiert, mit Giemsa (0,1 % Merck, Darmstadt, BRD) angefärbt, in PBS entfärbt und in Entellan (Merck, Darmstadt, BRD) eingebettet.

### Histologische Präparationen und Färbeverfahren

Verschiedene innere Organe (Gehirn, Herz, Lunge, Leber, Nieren, Milz und Gelenke) wurden von zuvor mit B. burgdorferi infizierten Mäusen zu unterschiedlichen Zeitpunkten nach der Infektion entfernt und entweder in flüssigem Stickstoff zur Präparation von Gefrierschnitten oder in 5 % Formaldehyd (in PBS) zur Einbettung in Paraffin oder Methacrylat aufbewahrt. Schnitte von 4 bis 7 µm wurden hergestellt, mit Hematoxylin-Eosin angefärbt und in Entellan (Merck AG, Darmstadt, BRD) eingebettet. Die Immunhistologie wurde unter Verwendung des Streptavidin-Biotin-Peroxidase-Systems durchgeführt (Kramer et al., (1989) Eur. J. Immunol. 19, 151).

Tabelle 1 zeigt, daß B. burgdorferi Organismen der Isolate ZS7 und B31 während der gesamten Versuchsdauer im Blut von Scid-Mäusen nachgewiesen wurden, die zuvor mit lebensfähigen Organismen geimpft wurden. Allerdings konnten nur Spirochäten vom Stamm ZS7, aber keine vom Stamm B31 in vitro rekultiviert werden. Bei Vergleich der rekultivierten Organismen mit dem primären B. burgdorferi ZS7 Isolat konnten keine Veränderungen im Proteingehalt oder im Plasmidprofil festgestellt werden. Keine oder nur äußerst geringe Titer an irrelevanten Antikörpern wurden in mit B. burgdorferi infizierten Scid-Mäusen während der gesamten Beobachtungsdauer nachgewiesen. In diesen Tieren wurden keine für B. burgdorferi spezifische IgM- oder IgG-Antikörper gefunden (Tabelle 1). Dagegen exprimierten alle C.B-17-Kontrollmäuse, die mit B. burgdorferi infiziert wurden, große Mengen an Gesamt-Ig und erhöhte Titer an für B. burgdorferi spezifischen IgM- und IgG-Antikörpern. Zwischen 7 und 20 Tagen nach der Infektion mit B. burgdorferi ZS7 zeigten die Scid-Mäuse erste klinische Symptome von Arthritis (Rötung und Schwellung beider Tibiotarsalgelenke), die mit der Zeit zunahmen. Dagegen wurden keine Symptome von Arthritis in Scid-Mäusen, die entweder mit UV-bestrahlten B. burgdorferi ZS7 oder mit lebensfähigen B. burgdorferi B31 Organismen infiziert wurden, und in C.B-17-Kontrollmäusen, die mit lebensfähigen B. burgdorferi ZS7 Organismen infiziert wurden, gefunden.

Auch histopathologisch wurden arthritische Gelenksveränderungen in Scid-Mäusen nachgewiesen, die mit lebensfähigen B. burgdorferi ZS7 infiziert wurden (Tabelle 1). Es wurden schwere Gelenkschädigungen festgestellt, gekennzeichnet durch die Anwesenheit von hyperplastisch entzündeten Synovial-Auskleidungszellen, verbunden mit Erosion und Zerstörung von Knorpelgewebe und/oder Knochen. Weiterhin wurde Pancarditis mit Infiltration von Mononuklearzellen in das Endocardium, Myocardium und Pericardium festgestellt. Ebenfalls wurde eine progressive Entzündung der Leber festgestellt, wobei eine Infiltration von Mononuklearzellen, die auf den Pfortaderbereich und die Zentralvene beschränkt war, granulomatöse Reaktionen und schließlich das Auftreten von Leberfibrose beobachtet wurde. Zusätzlich wurden geringere Schäden in den Nieren, der Lunge, dem Gehirn und der gestreiften Muskulatur festgestellt.

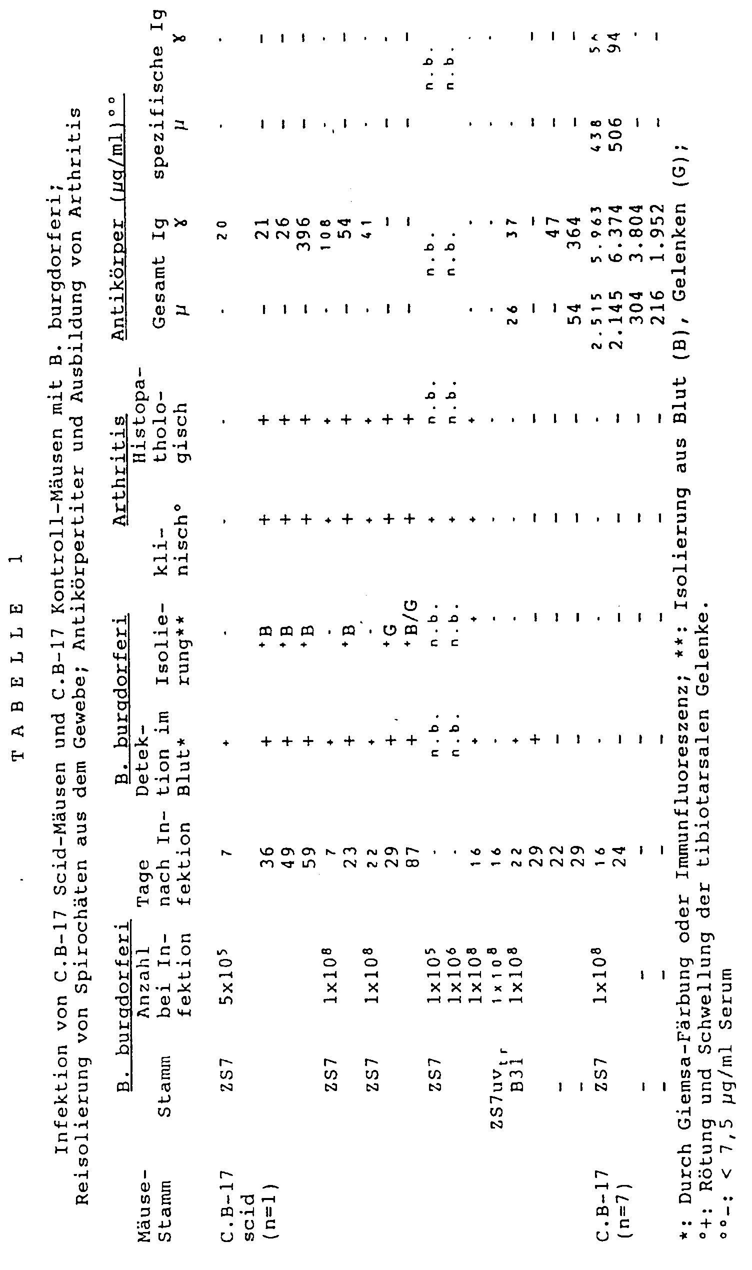

### Beispiel 2

### Wirkung eines für das B. burgdorferi 31kD Antigen spezifischen monoklonalen Antikörpers auf den Verlauf der Lyme-Borreliose in Scid-Mäusen

### Herstellung des monoklonalen Antikörpers

Bei Immunisierung einer Maus, die ein intaktes Immunsystem besitzt, mit B. burgdorferi Organismen werden polyklonale Antikörper exprimiert, die für B. burgdorferi spezifisch sind (siehe Tabelle 1).

Zehn Wochen alte weibliche Mäuse des Inzuchtstammes BALB/c wurden mit durch Beschallung homogenisierten Borrelien (B. burgdorferi, Stamm B31; ATCC 35 210) immunisiert.

### Immunisierungsprotokoll:

Tag 0: 200 µg Borrelien-Antigen in kompletten Freund's Adjuvants subkutan
Tag 21, 35, 49, 63: Challenge mit 100 µg Borrelien-Antigen in Phosphat-gepufferter Saline i.p.
Tag 66: Entnahme der Milz und Herstellung einer Einzelzellsuspension.

Die immunen Milzzellen wurden mit der Ag8-PAI Myelom-Zellinie nach Standardmethoden unter Verwendung von Polyethylenglycol fusioniert (J.H. Peters, H. Baumgarten, M. Schulze "Monoklonale Antikörper" Springer-Verlag, Heidelberg).

Die Fusionsprodukte wurden in 96 wells Gewebekulturplatten ausgesät. Nach 8 Tagen wurden die Zellkulturüberstände auf die Anwesenheit von B. burgdorferi spezifischen monoklonalen Antikörpern mit Hilfe eines Festphasen-ELISA, untersucht (J.H. Peters et al., s.o.).

Die Hybridoma-Zellen aus Antikörper-produzierenden Kulturen wurden nach der Grenzverdünnungsmethode kloniert. Die Kulturüberstände individueller Klone wurden anschließend erneut im Festphasen-ELISA sowie durch Western-Blot Analyse und durch Immunfluoreszenz-Untersuchungen charakterisiert. Der monoklonale Antikörper LA-2 der Subklasse IgG2b wird von einer monoklonalen Hybridoma-Linie produziert und sezerniert und reagiert im Western-Blot mit der 31kDa-Struktur, (Osp-A) von allen untersuchten B. burgdorferi Stämmen (u.a. die Isolate ZS7 und B31) bei Kontakt mit elektrophoretisch über ein SDS-Gel aufgetrennten und mittels Westernblot auf eine Membran transferierten B. burgdorferi Proteinen. Die monoklonalen Antikörper LA-26.1C(anti-ospA IgG1), LA 25.1 (anti-OspB (34 kDa Antigen); IgG2b) und LA 27.1 (anti-OspB (34 kDa Antigen), IgG1) wurden auf analoge Weise hergestellt und charakterisiert.

### Infektion von Mäusen mit B. burgdorferi ZS7

C.B-17 Scid-Mäuse wurden mit 1x10⁸ lebensfähigen B. burgdorferi ZS7-Organismen subkutan in die Schwanzwurzel infiziert.

### Behandlung der Mäuse mit Antiseren

Die infizierten Scid-Mäuse wurden zweimal pro Woche mit verschiedenen Antiseren behandelt. Die eine Gruppe wurde mit NMS (normales Mausserum), eine zweite Gruppe mit IMS (immunes Mausserum) und eine dritte Gruppe mit dem monoklonalen Antikörper LA-2 (gegen das 31 kD Antigen von B. burgdorferi) behandelt. Die Dosis der verabreichten Antiseren betrug in der ersten Woche 100 µl bzw. 100 µg bei LA-2, in der zweiten Woche 200 µl bzw. 200 µg bei LA-2 und in der dritten Woche 300 µl bzw. 300 µg bei LA-2.

Tabelle 2 zeigt, daß unbehandelte oder mit NMS behandelte Scid-Mäuse nach 12 Tagen klinische und histopathologische Anzeichen von Arthrithis bzw. Carditis und Hepatitis entwickelten. Dagegen bewirkte die Verabreichung des monoklonalen Antikörpers LA-2 bei Scid-Mäusen eine deutliche Reduzierung der Symptome. Klinisch waren nur leichte Rötungen der Gelenke und histopathologisch nur marginale Veränderungen festzustellen. Mit IMS behandelte Mäuse zeigten keine klinischen Arthrithisbefunde.

Ein Nachweis des B. burgdorferi Erregers durch in vitro Kultivierung gelang nur bei Mäusen, die entweder unbehandelt oder mit NMS behandelt wurden. Bei den mit LA-2 oder IMS behandelten Mäusen konnte B. burgdorferi nicht nachgewiesen werden (Tabelle 2).

**TABELLE 2**

| Behandlung von C.B-17 Scid-Mäusen mit B. burgdorferi und Antiseren | | | | | |
|---|---|---|---|---|---|
| Mäusestamm C.B-17 scid | Behandlung mit Antiserum | Arthritis (nach 12 Tagen) | | Carditis/Hepatitis histopathologisch | B. burgdorferi Nachweis (Anzucht) |
| | | klinisch | histopathologisch | | |
| n = 3 | - | + | + | + | + |
| n = 3 | NMS | + | + | + | + |
| n = 2 | IMS | - | - | - | - |
| n = 3 | LA-2 | -° | -°° | - | - |

| | | | | | |
|---|---|---|---|---|---|
| ° leichte Rötung des Gelenks | | | | | |
| °° nur marginale Veränderung | | | | | |

### Beispiel 3

### Expressionsklonierung des 31kD Antigens (OspA) von B. burgdorferi ZS7

### DNA-Präparation

Hochmolekulare DNA aus dem B. burgdorferi Stamm ZS7 wurde nach Kultivierung in modifiziertem Kelly's Medium gereinigt. Die Spirochäten wurden durch Zentrifugation bei 10.000 g pelletiert und dreimal in PBS-Puffer gewaschen. Das trockene Pellet wurde in 10 ml TE (10 mmol/l Tris, 1 mmol/l EDTA, pH 7,4) resuspendiert, mit Lysozym (5 mg/ml) 15 Minuten lang bei 30°C behandelt und die DNA durch Zugabe von 1 ml 20%igem SDS freigesetzt. Nach Zugabe von 1,5 ml NaCl (5 mol/l) wurde die Lösung mit einem gleichen Volumen an Phenol extrahiert, gefolgt von einer Extraktion mit Chloroform. Die DNA wurde dann durch Zugabe von 2 Volumina absolutem Ethanol und Inkubation bei -20°C über Nacht gefällt. Nach Zentrifugation wurde der Rückstand in 0,5 ml TE gelöst und mit DNAse freier RNAse A (20 µg/ml) 45 Minuten lang bei 55°C inkubiert, gefolgt von einer einstündigen Behandlung mit Proteinase K (0,1 µg/ml) bei 37°C. Die Lösung wurde auf 0,3 mol/l NaOAc eingestellt und mit Phenol-Chloroform wie oben beschrieben extrahiert. Nach Fällung mit Ethanol wurde die DNA wieder in TE aufgenommen.

### Herstellung der Genbank

Hochmolekulare DNA wurde durch drei Sekunden lange Ultraschallbehandlung statistisch zerkleinert. T4-DNA-Polymerase (30 Minuten bei 37°C) und Klenow-Enzym (5 Minuten bei 20°C) wurden dazu verwendet, um die Enden der erzeugten DNA-Fragmente zu glätten. DNA mit glatten Enden wurde in die BamHI-Stelle eines Expressionsvektors pUEX1 unter Verwendung einer Adapter-Klonierungsstrategie ligiert (Bresan und Stanley (1987) Nucl. Acid. Res. S. 1056). Nach einem Größenselektionsschritt durch eine Molekularsieb-Chromatographie über Sephacryl S-1000 und Transformation von kompetenten Wirtszellen E. coli (MC 1061) wurde der Anteil an rekombinanten Plaque bildenden Einheiten (pfu) wie folgt bestimmt: zufällig ausgewählte Kolonien wurden gepickt und bis zur Sättigung in 2 ml Selektionsmedium (LB mit 25 µg/ml Ampicillin) angezüchtet. Die Plasmid-DNA wurde nach der üblichen alkalischen Lysis-Methode isoliert und anschließend mit BamHI geschnitten. Mehr als 50 % der analysierten Plasmide enthielten durchschnittlich ≥ 1,5 kb lange DNA-Insertionen.

### Ausplattieren und Screening der B. burgdorferi ZS7-Genbank

Die Zellen wurden auf 24x24 cm Platten bei einer Dichte von 7.000 pfu pro Platte ausplattiert und über Nacht bei 30°C inkubiert. Nach dem Transfer der Kolonien auf Nitrocellulosefilter (NC) wurde die Expression von β-Galactosidase-Fusionsproteinen durch zweistündige Inkubation bei 42°C induziert. Die Filter wurden auf ein Whatman 3MM-Papier transferiert, das mit 5 % SDS behandelt worden war, und etwa 25 Minuten lang bei 95°C inkubiert. Dann wurden die Proteine elektrogeblottet unter Verwendung einer üblichen Apparatur zum halbtrockenen Westernblotting. Nach DNAse-Behandlung der NC-Filter wurden immunreaktive Klone durch ein Expressions-Screening unter Verwendung monoklonaler Antikörper identifiziert. Unspezifische Bindungsstellen auf den NC-Filtern wurden durch vierstündige Inkubation mit PBS, enthaltend 0,2 % (Gewicht pro Volumen) Gelatine und 3 mmol/l NaN₃ bei Raumtemperatur abgesättigt. Anschließend wurden die Filter mit Kulturüberständen des Anti-31 kD monoklonalen Antikörperklons LA-2 18 Stunden lang unter andauerndem Schütteln inkubiert. Nach gründlichem Waschen (PBS + 1 % (Volumen/Volumen) Triton X-100; PBS + 0,5 mol/l Natriumchlorid; PBS + 1 mol/l Natriumchlorid; jeder Schritt 10 Minuten) wurden die Filter mit der 1:10.000 Verdünnung einer Peroxidase-markierten F(ab)₂-Präparation von Kaninchen-Anti-Maus-IgG-Antikörpern 1,5 Stunden lang bei Raumtemperatur unter andauerndem Schütteln inkubiert. Die Filter wurden wieder, wie oben beschrieben, gewaschen und dann mit Diaminobenzidin als Peroxidasesubstrat inkubiert. Von 10⁴ rekombinanten PFU's reagierten 20 Klone mit dem monoklonalen Antikörper LA-2.

### Sequenzanalyse des 31 kD Antigens (OspA)

Die insertierte DNA eines rekombinanten E. coli Klons mit positiver Antikörperreaktion mit LA-2 wurde auf übliche Weise isoliert. Die DNA-Insertion dieses Klons enthielt das für das B. burgdorferi 31 kD Antigen kodierende ospA-Gen in voller Länge. Das Plasmid, das die Insertion enthält, wurde pZS-7/31-2 bezeichnet und wurde gemäß Budapester Vertrag bei DSM (unter der Nummer DSM 5528) hinterlegt.

Das von diesem immunpositiven Klon produzierte rekombinante Protein wurde als rZS7/31-2 bezeichnet. Die kodierende DNA-Sequenz des ospA-Gens wurde bestimmt. Sie ist zusammen mit der davon abgeleiteten Aminosäuresequenz des OspA Proteins in Abb. 1 dargestellt.

Aus Abb. 1 ist auch ersichtlich, daß das 31 kD Antigen von B. burgdorferi einem Protein mit 273 Aminosäuren entspricht.

### Präparation von Nicht-Fusionsproteinen

a) Der Klon, der das immunreaktive Protein rZS7/31-2 exprimiert, wurde über Nacht bei 30°C in 10 ml LB mit Ampicillin gezüchtet. 1 ml der Kultur wurde in 100 ml Selektionsmedium eingebracht und bei 30°C mit guter Belüftung bis zu einer Dichte von 8x10⁷ Zellen pro ml (A600=0,2) gezüchtet. Die Expression des rekombinanten Proteins wurde durch einen Transfer der Wirtszellen auf 42°C erreicht. Nach Abkühlen und Zentrifugation wurden die Zellen in STE-Puffer (10 mmol/l Tris, 100 mmol/l Natriumchlorid, 1 mmol/l EDTA, pH 8,0) gewaschen und der Rückstand in 0,6 ml Lysispuffer (25 % Sucrose, 50 mmol/l Tris, pH 8,0) resuspendiert. Nach Zugabe von 150 µl Lysozym (10 mg/ml) wurde die Mischung 15 Minuten lang auf Eis inkubiert, gefolgt von einer weiteren Inkubation (15 Minuten auf Eis) mit 18 µl DNAse 1 (10 mg/ml) in Gegenwart von 5 µl 1 mol/l Magnesiumchlorid. Schließlich wurden 250 µl 4x Detergensmix (1 % Triton X 100, 0,5 % Deoxycholat, 0,1 mol/l NaCl, 10 mmol/l Tris, pH 7,4) zugefügt und 5 Minuten lang auf Eis inkubiert. Nach Zentrifugation wurde der Rückstand zweimal mit Puffer A (50 mmol/l Tris, 50 mmol/l NaCl, 1 mmol/l EDTA, pH 8,0) gewaschen und in 9 Volumina Puffer A, zusätzlich 8 M Harnstoff enthaltend, resuspendiert und eine Stunde lang bei Raumtemperatur inkubiert. Die Probe wurde verdünnt mit 9 Teilen Puffer B (50 mmol/l KH₂PO₄/K₂HPO₄, 50 mol/l NaCl, 1 mmol/l EDTA, pH 10,7) und 30 Minuten lang bei Raumtemperatur gerührt, wobei der pH-Wert durch Zugabe von KOH bei 10,7 gehalten wurde. Nach Einstellung eines pH-Werts der Lösung auf 7,0 durch Zugabe von HCl wurde die Probe über Nacht gegen Puffer A dialysiert (bei 4°C) und 10 Minuten lang bei 4°C und 10.000 Umläufen pro Minute (Upm) in einem SS34-Rotor zentrifugiert. Der Überstand, der das rekombinante Protein enthält, wurde bei -20°C aufbewahrt.
b) Da der Klon das imunreaktive Protein rZS7/31-2 auch in das Kulturmedium sezerniert, ist eine Reinigung (Affinitätchromatographie) direkt aus Kulturüberstand möglich.

### Präparation von rekombinanten OspA (Nicht-Fusions) Protein und Affinitäts-chromatografische Reinigung

Die rekombinanten Proteine wurden anschließend Affinitäts-chromatographisch gereinigt. Zu diesem Zweck wurden gereinigte monoklonale Antikörper LA-2 an aktivierte Sepharose CL 4B kovalent gebunden. Der dialysierte Harnstoff-Extrakt mit dem rekombinanten Protein wurde an Maus IgG-Sepharose CL 4B adsorbiert und anschließend über die LA-2-Sepharose CL 4B Säule gegeben. Nach intensivem Waschen wurde das gebundene rekombinante Protein mit 0,1 mol/l Glycin/HCl - 0,1 mol/l NaCl, pH 2,5 eluiert. Der pH-Wert der gesammelten Fraktionen wurde durch sofortige Zugabe von 1/10 Vol. 0,5 mol/l K₂HPO₄ neutralisiert. Die proteinhaltigen Fraktionen wurden konzentriert und dialysiert. Mit Hilfe der SDS-Polyacrylamid Gel Elektrophorese wurde der Grad der Reinigung bestimmt.

### Immunologische Charakterisierung des rekombinanten Proteins rZS7/31-2

Das rekombinante Protein rZS7/31-2 wurde immunologisch untersucht. Zum Vergleich wurde das rekombinante Protein rB31/41-9 (B. burgdorferi 41 kD Oberflächenantigen) herangezogen.

Flachboden-Mikrotiterplatten wurden mit Harnstoffextrakten der rekombinanten Proteine rZS7/31-2 und rB31/41-9 bzw. einem Harnstoffextrakt des zur Genexpression verwendeten E. coli Stamms MC 1061 beschichtet. Unspezifische Bindungsstellen wurden mit 0,2 % Gelatine in phosphatgepufferter Kochsalzlösung blockiert. Vertiefungen der so vorbereiteten Mikrotiterplatten wurden mit den angegebenen monoklonalen Antikörpern LA-2 (anti-31 kD, Osp-A), LA-1 (anti-41 kD, Flagellin) bzw. ACHT-2 (anti-α₁-Antichymotrypsin) in Ansatz gebracht.

Die gebundenen monoklonalen Antikörper wurden mit peroxidasemarkierten speziesspezifischen anti-Maus Immunglobulinen zur Reaktion gebracht. Gebundene peroxidasemarkierte Antikörper wurden unter Verwendung des Peroxidasesubstrats Orthophenylendiamin quantifiziert. Die Absorption bei 492 nm (A₄₉₂) wurde direkt in der Mikrotiterplatte mit Hilfe eines automatisierten Plattenphotometers bestimmt. Die Stärke der Absorption ist ein Maß für die Menge gebundener monoklonaler Antikörper.

Der monoklonale Antikörper LA-2 reagiert in spezifischer Weise mit rZS7/31-2 aber nicht mit MC 1061 bzw. rB31/41-9. Die Kontrollreaktion des monoklonalen Antikörpers LA-1 ist spezifisch für rB31/41-9. Der monoklonale Kontrollantikörper ACHT-2 (Negativkontrolle) zeigt auf keinem der Proteine eine signifikante Reaktion.

Abbildung 2 zeigt, daß das vom monoklonalen Antikörper LA-2 in spezifischer Weise erkannte antigene Epitop auf dem rekombinanten Protein rZS7/31-2 exprimiert ist, das aus dem Genom von B. burgdorferi ZS7 kloniert wurde.

### Beispiel 4

### Vergleich von für das 31 kD (OspA) bzw. 34 kD Antigen (OspB) spezifischen Antikörpern mit Antikörpern, die für das 41 kD Antigen (Flagellin) spezifisch sind

Die monoklonalen Antikörper LA-2 und LA-26.1 erkennen das 31 kD Antigen OspA und sind vom Isotyp IgG2b bzw. IgG1. Die monoklonalen Antikörper LA-25.1 und LA-27.1 erkennen das 34 kD Antigen OspB und sind vom Isotyp IgG2b bzw. IgG1. Die monoklonalen Antikörper LA-10 und LA-21 sind spezifisch für das Flagellen-assoziierte 41 kD periplasmatische Protein von B. burgdorferi und sind vom Isotyp IgG2a bzw. IgG1. Alle obengenannten Antikörper wurden nach dem in Beispiel 2 beschriebenen Verfahren erhalten. Es sollte in diesem Versuch festgestellt werden, ob auch monoklonale Antikörper gegen ein anderes B. burgdorferi Antigen in Scid-Mäusen eine Protektion vor den klinischen Symptomen der Lyme-Borreliose bewirken.

Das polyklonale Anti-B31-Immunserum (IMS) wurde aus C57BL/6-Mäusen 91 Tage nach einer subkutanen Inokkulation mit 1x10⁸ B. burgdorferi B31-Organismen entnommen. Das polyklonale Anti-ZS7 IMS wurde aus C57BL/6-Mäusen 68 Tage nach einer subkutanen Inokulation mit 1x10⁸ B. burgdorferi ZS7 entnommen. Beide Sera enthielten 60 µg/ml spezifische Antikörper, wie in einem ELISA-System bestimmt wurde (Schaible et al., J. Exp. Med. 170 (1989), 1427-1432). Das Normal-Maus-Serum (NMS) wurde aus nicht infizierten C57BL/6-Mäusen entnommen.

Zum Zeitpunkt der Inokulation und darauffolgend in 4-Tages-Intervallen wurden die angegebenen Antikörper, das IMS, das NMS oder PBS-Puffer passiv intraperitoneal in Scid-Mäuse gemäß folgendem Protokoll transferiert:
Tag 0 und Tag 3: 100 µl,
Tag 7 und Tag 10: 200 µl,
Tag 13 und Tag 17: 300 µl.

Scid-Mäuse, die entweder mit Anti-ZS7IMS, Anti-B31IMS oder mit dem monoklonalen Antikörper LA-2 behandelt wurden, zeigten keine sichtbaren klinischen Symptome von Arthritis, d.h. es trat keine Rötung und Schwellung von Tibioltarsalgelenken während der 21 Beobachtungstage auf. Ebenso waren keine Symptome von Carditis und Hepatitis festzustellen. Histopathologische Untersuchungen ergaben keine Veränderungen in den Gelenken, dem Herzen und der Leber von Scid-Mäusen, die entweder mit Anti-ZS7-IMS, Anti-B31-IMS oder mit dem monoklonalen Antikörper LA-2 behandelt wurden.

Auch der andere OspA-spezifische monoklonale Antikörper LA-26.1 vom Isotyp IgG1 sowie die OspB spezifischen Antikörper LA-25.1 und LA-27.1 waren in der Lage, die klinischen Symptome von Arthritis, Carditis und Hepatitis zu mildern. Hier zeigten sich leichte pathologische Veränderungen in den untersuchten Organen.

Im Gegensatz dazu zeigten Scid-Mäuse, die entweder PBS-Puffer, NMS oder monoklonale Antikörper gegen Flagellin (LA-10 oder LA-21) verabreicht bekommen hatten, klinische Zeichen von Arthritis, die für unbehandelte Scid-Mäuse typischen, pathologischen Veränderungen (Tabelle 3). Die Schwere der Symptome in den letztgenannten Tieren war mit zunehmender Zeitdauer nach der Inokkulation ansteigend und schwächte sich während der gesamten Beobachtungsperiode nicht ab. Aus Scid-Mäusen, die zuvor entweder mit Anti-ZS7IMS oder mit dem Antikörper LA-2 behandelt wurden, konnten keine Spirochäten isoliert werden. Im Gegensatz dazu war der Nachweis von Spirochäten durch Immunfluoreszenz und durch Kultivierung aus dem Blut von Scid-Mäusen möglich, die mit PBS-Puffer, NMS oder den monoklonalen Antikörpern LA-25.1, LA-26.1, LA-27.1, LA-10 oder LA-21 behandelt worden waren.

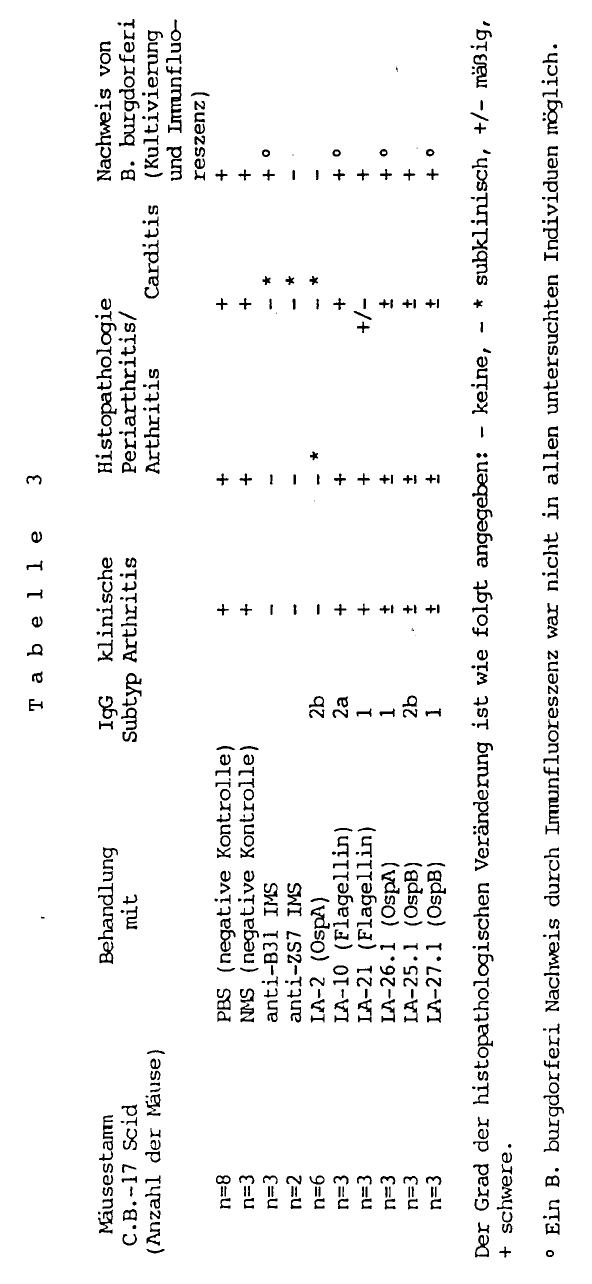

### Beispiel 5

### Wirkung von Antiserum aus, mit OspA immunisierten Mäusen auf den Verlauf der Lyme-Borreliose in Scid-Mäusen

In diesem Experiment konnte gezeigt werden, daß eine Verabreichung von nativem OspA (isoliert aus B. burgdorferi ZS-7) oder rekombinantem OspA (isoliert aus E. coli Bakterien , die mit dem rekombinanten Plasmid pZS7/31-2 (DSM 5528) transformiert waren) in normalen Mäusen (Mäusestamm C57BL/6) die Bildung von protektiven polyklonalen Antikörpern induziert. Werden diese Antikörper Scid-Mäusen verabreicht, wird ein Schutz gegen die Lyme-Borreliose bewirkt. Dabei wird festgestellt, daß rekombinantes OspA eine mit nativem OspA vergleichbare protektive Immunantwort induziert. Das Ergebnis und die Details der Versuchsdurchführung bei diesem Experiment sind in Tabelle 4 angegeben.

Die Gewinnung von rekombinanten OspA ist in Beispiel 3 angegeben.

Die Gewinnung von nativem OspA sowie die Immunisierung der Mäuse mit OspA geschah wie folgt:

### Anreicherung von nativem 31kDa OspA

3,2x10¹⁰ Spirochäten werden für zwei Stunden bei 4°C in 5 ml PBS/7,5 ml n-Butanol in Gegenwart von Proteaseinhibitoren (5mmol/l EDTA, 5mmol/l Benzamidin und 0,5mmol/l PMSF) auf dem Magnetrührer gerührt. Danach wird das Gemisch für 90 Minuten bei 10000 rpm und 4°C in der Sorvall-Zentrifuge (Festwinkelrotor) zentrifugiert. Die wäßrige Phase, die die Oberflächenproteine enthält, wird abgenommen und dreimal mit Chloroform gewaschen.

Der Proteingehalt wird über die Extinktion bei 280 nm bzw. mit dem BCA-Test bestimmt.

Im Silbergel bzw. Westernblot mit anti-B. burgdorferi Kaninchenserum wurden für Stamm ZS7 eine Hauptbande im Molekulargewichtsbereich von 31 kDa sowie schwache Banden bei 20, 34 und 65-68 kDa gefunden. Die Butanol/Wasser Präparation von B. burgdorferi Stamm B31 ergab eine Hauptbande bei 31 kDa, sowie schwache Banden bei 20 und 34 kDa.

### Immunisierung von Mäusen mit nativem und rekombinanten OspA

C57BL6 bzw. C.B-17 Mäusen wurde 3x im Abstand von 7 bis 10 Tagen 5 µg (natives OspA von Stamm B31) bzw. 10 µg (natives OspA von Stamm ZS7, rekombinantes OspA von ZS7) in 100 µl Adjuvant (ABM3; Fa. Sebak, Aidenbach, BRD) subkutan in die Schwanzwurzel gegeben. Frühestens 3 Wochen nach der letzten Immunisierung konnte für 3 bis 4 Monate Serum abgenommen werden. Der Gehalt an spezifischen Antikörpern wird im ELISA-System bestimmt.

**Tabelle 4**

| Effekt von B. burgdorferi-spezifischen monoklonalen und polyklonalen Antikörpern auf die Spirochätose und Entwicklung der Arthritis in mit B. burgdorferi infizierten Scid Mäusen | | | | | |
|---|---|---|---|---|---|
| Mäusestamm C.B.-17 Scid (Anzahl der Mäuse) | Behandlung mit | Entwicklung von Arthritis nach Woche(n) | | | Nachweis von B. burgdorferi |
| | | 1 | 2 | 3 | |
| n = 6 | PBS (negative Kontrolle) | ± | + | + | 6/6 |
| n = 6 | LA-2 | - | - | - | 0/3 |
| n = 2 | anti OspA (nativ) IMS | - | - | - | 0/2 |
| n = 3 | anti OspA (recomb.) IMS | - | - | - | 0/3 |
| Erster Antikörpertransfer i.p. (100µl) Tag 0 (Tag der Inokulation mit B. burgdorferi ZS-7, 1x10³ Organismen, s.c. in die Schwanzwurzel). Weitere Antikörpertransfers Tag 4 (100µl), Tag 7 (200µl), Tag 11 (200µl), Tag 14 (300µl), Tag 18 (300µl), (i.p.) | | | | | |

SEQUENZPROTOKOLL

SEQ ID No: 1

ART DER SEQUENZ:
Nukleotid mit entsprechendem Protein

SEQUENZLÄNGE: | 822 Nukleotide
bzw. 273 Aminosäuren

STRANGFORM: | Einzelstrang

TOPOLOGIE: | linear

ART DES MOLEKÜLS: | Genom-DNA

URSPRÜNGLICHE HERKUNFT

ORGANISMUS: | Borrelia burgdorferi ZS7
(DSM 5527)

UNMITTELBARE EXPERIMENTELLE HERKUNFT

NAME DES PLASMIDS: | pZS-7/31-2 (DSM 5528)

EIGENSCHAFTEN: | 31 kDa Antigen (OspA)
aus B. burgdorferi ZS7

ATG AAA AAA TAT TTA TTG GGA ATA GGT CTA 30
Met Lys Lys Tyr Leu Leu Gly Ile Gly Leu
                5                   10
ATA TTA GCC TTA ATA GCA TGT AAG CAA AAT 60
Ile Leu Ala Leu Ile Ala Cys Lys Gln Asn
                15                  20
GTT AGC AGC CTT GAC GAG AAA AAC AGC GTT 90
Val Ser Ser Leu Asp Glu Lys Asn Ser Val
                25                  30
TCA GTA GAT TTG CCT GGT GAA ATG AAC GTT 120
Ser Val Asp Leu Pro Gly Glu Met Asn Val
                35                  40
CTT GTA AGC AAA GAA AAA AAC AAA GAC GGC 150
Leu Val Ser Lys Glu Lys Asn Lys Asp Gly
                45                  50
AAG TAC GAT CTA ATT GCA ACA GTA GAC AAG 180
Lys Tyr Asp Leu Ile Ala Thr Val Asp Lys
                55                  60
CTT GAG CTT AAA GGA ACT TCT GAT AAA AAC 210
Leu Glu Leu Lys Gly Thr Ser Asp Lys Asn
                65                  70
AAT GGA TCT GGA GTA CTT GAA GGC GTA AAA 240
Asn Gly Ser Gly Val Leu Glu Gly Val Lys
                75                  80
GCT GAC AAA AGT AAA GTA AAA TTA ACA ATT 270
Ala Asp Lys Ser Lys Val Lys Leu Thr Ile
                85                  90
TCT GAC GAT CTA GGT CAA ACC ACA CTT GAA 300
Ser Asp Asp Leu Gly Gln Thr Thr Leu Glu
                95                  100
GTT TTC AAA GAA GAT GGC AAA ACA CTA GTA 330
Val Phe Lys Glu Asp Gly Lys Thr Leu Val
                105                 110
TCA AAA AAA GTA ACT TCC AAA GAC AAG TCA 360
Ser Lys Lys Val Thr Ser Lys Asp Lys Ser
                115                 120

TCA ACA GAA GAA AAA TTC AAT GAA AAA GGT 390
Ser Thr Glu Glu Lys Phe Asn Glu Lys Gly
                125                 130
GAA GTA TCT GAA AAA ATA ATA ACA AGA GCA 420
Glu Val Ser Glu Lys Ile Ile Thr Arg Ala
                135                 140
GAC GGA ACC AGA CTT GAA TAC ACA GAA ATT 450
Asp Gly Thr Arg Leu Glu Tyr Thr Glu Ile
                145                 150
AAA AGC GAT GGA TCT GGA AAA GCT AAA GAG 480
Lys Ser Asp Gly Ser Gly Lys Ala Lys Glu
                155                 160
GTT TTA AAA AGC TAT GTT CTT GAA GGA ACT 510
Val Leu Lys Ser Tyr Val Leu Glu Gly Thr
                165                 170
TTA ACT GCT GAA AAA ACA ACA TTG GTG GTT 540
Leu Thr Ala Glu Lys Thr Thr Leu Val Val
                175                 180
AAA GAA GGA ACT GTT ACT TTA AGC AAA AAT 570
Lys Glu Gly Thr Val Thr Leu Ser Lys Asn
                185                 190
ATT TCA AAA TCT GGG GAA GTT TCA GTT GAA 600
Ile Ser Lys Ser Gly Glu Val Ser Val Glu
                195                 200
CTT AAT GAC ACT GAC AGT AGT GCT GCT ACT 630
Leu Asn Asp Thr Asp Ser Ser Ala Ala Thr
                205                 210
AAA AAA ACT GCA GCT TGG AAT TCA GGC ACT 660
Lys Lys Thr Ala Ala Trp Asn Ser Gly Thr
                215                 220
TCA ACT TTA ACA ATT ACT GTA AAC AGT AAA 690
Ser Thr Leu Thr Ile Thr Val Asn Ser Lys
                225                 230
AAA ACT AAA GAC CTT GTG TTT ACA AAA GAA 720
Lys Thr Lys Asp Leu Val Phe Thr Lys Glu
                235                 240

AAC ACA ATT ACA GTA CAA CAA TAC GAC TCA 750
Asn Thr Ile Thr Val Gln Gln Tyr Asp Ser
                245                 250
AAT GGC ACC AAA TTA GAG GGG TCA GCA GTT 780
Asn Gly Thr Lys Leu Glu Gly Ser Ala Val
                255                 260
GAA ATT ACA AAA CTT GAT GAA ATT AAA AAC 810
Glu Ile Thr Lys Leu Asp Glu Ile Lys Asn
                265                 270
GCT TTA AAA TAA
Ala Leu Lys -

## Patentansprüche (Patentansprüche für folgende(n) Vertragsstaat(en): AT, BE, CH, DE, DK, FR, GB, IT, LI, LU, NL, SE)

1. Verfahren zur Gewinnung eines Impfstoffs gegen die Lyme-Krankheit,
**dadurch gekennzeichnet,**
daß man Versuchstiere mit dem 31 kD Antigen (OspA) oder/und mit dem 34 kD Antigen (OspB) von B.burgdorferi ZS7 (DSM 5527) immunisiert und aus dem immunisierten Versuchstier auf übliche Weise protektive, polyklonale oder monoklonale Antikörper gegen OspA oder/und OspB gewinnt.

2. Verfahren nach Anspruch 1,
**dadurch gekennzeichnet,**
daß man Mäuse als Versuchstiere verwendet.

## Patentansprüche (Patentansprüche für folgende(n) Vertragsstaat(en): ES, GR)

1. Verfahren zur Gewinnung eines Impfstoffs gegen die Lyme-Krankheit,
**dadurch gekennzeichnet,**
daß man Versuchstiere mit dem 31 kD Antigen (OspA) oder/und mit dem 34 kD Antigen (OspB) von B.burgdorferi immunisiert und aus dem immunisierten Versuchstier auf übliche Weise protektive, polyklonale oder monoklonale Antikörper gegen OspA oder/und OspB gewinnt.

2. Verfahren nach Anspruch 1,
**dadurch gekennzeichnet,**
daß man Mäuse als Versuchstiere verwendet.

3. Verfahren nach Anspruch 1 oder 2,
**dadurch gekennzeichnet,**
daß man das OspA Antigen oder/und das OspB Antigen aus B.burgdorferi ZS7 (DSM 5527) verwendet.

## Claims (Claims for the following Contracting State(s): AT, BE, CH, DE, DK, FR, GB, IT, LI, LU, NL, SE)

1. Process for producing a vaccine against Lyme disease,
**wherein**
experimental animals are immunized with the 31 kD antigen (OspA) or/and with the 34 kD antigen (OspB) of B. burgdorferi ZS7 (DSM 5527) and protective, polyclonal or monoclonal antibodies against OspA or/and OspB are isolated in the usual manner from the immunized experimental animal.

2. Process as claimed in claim 1,
**wherein**
mice are used as experimental animals.

## Claims (Claims for the following Contracting State(s): ES, GR)

1. Process for producing a vaccine against Lyme disease,
**wherein**
experimental animals are immunized with the 31 kD antigen (OspA) or/and with the 34 kD antigen (OspB) of B. burgdorferi and protective, polyclonal or monoclonal antibodies against OspA or/and OspB are isolated in the usual manner from the immunized experimental animal.

2. Process as claimed in claim 1,
**wherein**
mice are used as experimental animals.

3. Process as claimed in claim 1 or 2,
**wherein**
the OspA antigen or/and the OspB antigen from B. burgdorferi ZS7 (DSM 5527) is used.

## Revendications (Revendications pour l'(les) Etat(s) contractant(s) suivant(s): AT, BE, CH, DE, DK, FR, GB, IT, LI, LU, NL, SE)

1. Procédé de production d'un vaccin contre la maladie de Lyme, caractérisé en ce que l'on immunise des animaux expérimentaux avec l'antigène de 31 kD (OspA) et/ou avec l'antigène de 34 kD (OspB) de *B. burgdorferi* ZS7 (DSM 5527) et on récupère de manière classique à partir de l'animal expérimental immunisé des anticorps protecteurs, polyclonaux ou monoclonaux, dirigés contre l'OspA et/ou l'OspB.

2. Procédé selon la revendication 1, caractérisé en ce que l'on utilise des souris comme animaux expérimentaux.

## Revendications (Revendications pour l'(les) Etat(s) contractant(s) suivant(s): ES, GR)

1. Procédé de production d'un vaccin contre la maladie de Lyme, caractérisé en ce que l'on immunise des animaux expérimentaux avec l'antigène de 31 kD (OspA) et/ou avec l'antigène de 34 kD (OspB) de *B. burgdorferi* et on récupère de manière classique à partir de l'animal expérimental immunisé des anticorps protecteurs, polyclonaux ou monoclonaux, dirigés contre l'OspA et/ou l'OspB.

2. Procédé selon la revendication 1, caractérisé en ce que l'on utilise des souris comme animaux expérimentaux.

3. Procédé selon la revendication 1 ou 2, caractérisé en ce que l'on utilise l'antigène OspA et/ou l'antigène OspB de *B. burgdorferi* ZS7 (DSM 5527).
